# FASCICULE DE BREVET EUROPEEN

(11) **EP 1 060 754 B1**
(45) Date de publication et mention de la délivrance du brevet: **24.08.2005**
(21) Numéro de dépôt: 00401684.6
(22) Date de dépôt: 14.06.2000
(51) Int. Cl.: A61M 5/14

(54) **Kit de perfusion de poches multiples**
Infusionsystem mit mehreren Infusionsbeuteln
Infusion system with several bags

(30) Priorité: 18.06.1999 FR 9907773
(43) Date de publication de la demande: 20.12.2000
(73) Titulaire: MACO PHARMA, F-59200 Tourcoing (Nord) (FR)
(72) Inventeur: Demay, Sylvie, 59200 Tourcoing (FR); Verpoort, Thierry, 59420 Mouvaux (FR); Goudaliez, Francis, 59155 Faches Thumesnil (FR)
(74) Mandataire: Derambure, Christian

(56) Documents cités:
- WO-A-92/11881
- GB-A- 116 351
- US-A- 4 447 230
- US-A- 4 512 764
- US-A- 4 838 858

## Description

L'invention est relative aux systèmes de perfusion d'un médicament.

On connaît déjà des systèmes de perfusion du type comprenant une poche destinée à contenir un médicament et des moyens de distribution et de perfusion de celui-ci.

Pour des raisons de commodité, les moyens de distribution et de perfusion ont une longueur relativement importante et une contenance non négligeable par rapport volume de la poche.

Par conséquent, lorsque la poche est vide, une quantité non négligeable de médicament reste dans les moyens de distribution et de perfusion et n'est donc pas perfusée au patient.

Pour cette raison, il est préférable de rincer les moyens de distribution et de perfusion avec le contenu d'une seconde poche qui est connectée aux moyens de distribution à la place de la poche contenant le médicament.

Toutefois, cette formule ne donne pas parfaitement satisfaction parce qu'elle implique la déconnexion de la ligne de perfusion,- c'est à dire son ouverture- ce qui implique un risque de contamination.

On connaît du document WO-92/11881 un système de rinçage d'un site d'injection IV qui comprend, en circuit fermé, deux réservoirs contenant chacun une solution de rinçage, et un ensemble de tubulures pour connecter lesdits réservoirs au site d'injection IV. Le système de rinçage comprend un connecteur sur lequel le patient vient connecter un appareil de perfusion pour injecter un médicament, ce qui pose également des risques de contamination.

L'invention vise donc à remédier à ces inconvénients.

A cet effet, et selon un premier aspect, elle propose un système de perfusion comprenant au moins une poche - dite poche de médicament - destinée à contenir un certain produit tel qu'un médicament en solution dans un soluté, et des moyens de distribution et de perfusion associés aux dites poches dans lequel au moins une poche - dite poche de rinçage - destinée à contenir un soluté pouvant servir de soluté de rinçage est associée audit système de manière que le soluté de rinçage puisse passer dans les moyens de distribution et de perfusion afin d'assurer leur rinçage ; et comprenant en outre des moyens de sélection à commande externe, notamment manuelle, aptes à sélectionner le passage dans les moyens de distribution et de perfusion du soluté contenant le médicament et/ou du soluté de rinçage ; les poches, les moyens de distribution et de perfusion et les moyens de sélection formant un ensemble unitaire en circuit fermé, en permanence.

Suivant une première réalisation, la poche de rinçage est associée par l'intermédiaire d'une tubulure aux moyens de distribution et de perfusion.

Dans une première variante, les moyens de sélection comprennent un clamp positionné sur la tubulure associant la poche de rinçage aux moyens de distribution et de perfusion.

Dans une seconde variante, les moyens de sélection comprennent un robinet à voies multiples réalisant la connexion entre la tubulure associant la poche de rinçage et les moyens de distribution et de perfusion.

Les moyens de sélection comprennent en outre des systèmes sécables aptes, lors de leur rupture, à mettre la poche de rinçage et/ou les poches de médicaments en communication fluidique avec les moyens de distribution et de perfusion.

Suivant une autre réalisation, la poche de rinçage est associée directement sur une poche de médicament et les moyens de sélection comprennent un système sécable apte, lors de sa rupture, à mettre en communication fluidique la poche de rinçage avec la poche de médicament.

Le système de perfusion peut comprendre au moins deux poches de médicament destinées à recevoir deux doses de médicament identiques ou différentes, et au moins une poche de rinçage, et en outre un moyen de commutation de la communication entre les poches de médicament et les moyens de distribution et de perfusion.

Selon un deuxième aspect, l'invention concerne un procédé de mise en oeuvre d'un système de perfusion décrit ci-dessus, dans lequel, après avoir perfusé le contenu de la poche de médicament, on manoeuvre les moyens de sélection pour permettre le rinçage des moyens de distribution et de perfusion grâce au soluté de rinçage provenant de la poche de rinçage.

Dans un mode d'exécution particulier, l'invention concerne un procédé pour la mise en oeuvre d'un système de perfusion à deux poches de médicament et une poche de rinçage, dans lequel, après avoir perfusé le contenu de la première poche de médicament, on manoeuvre les moyens de sélection pour permettre le rinçage des moyens de distribution et de perfusion avec une partie du contenu de la poche de rinçage, puis on manoeuvre les moyens de sélection et les moyens de commutation pour perfuser le contenu de la deuxième poche de médicament, enfin, lorsque cette deuxième poche est vide, on manoeuvre les moyens de sélection pour permettre le rinçage des moyens de distribution et de perfusion avec le reste du contenu de la poche de rinçage.

L'invention sera bien comprise grâce à la description qui suit en référence au dessin annexé, illustrant divers modes de réalisation.

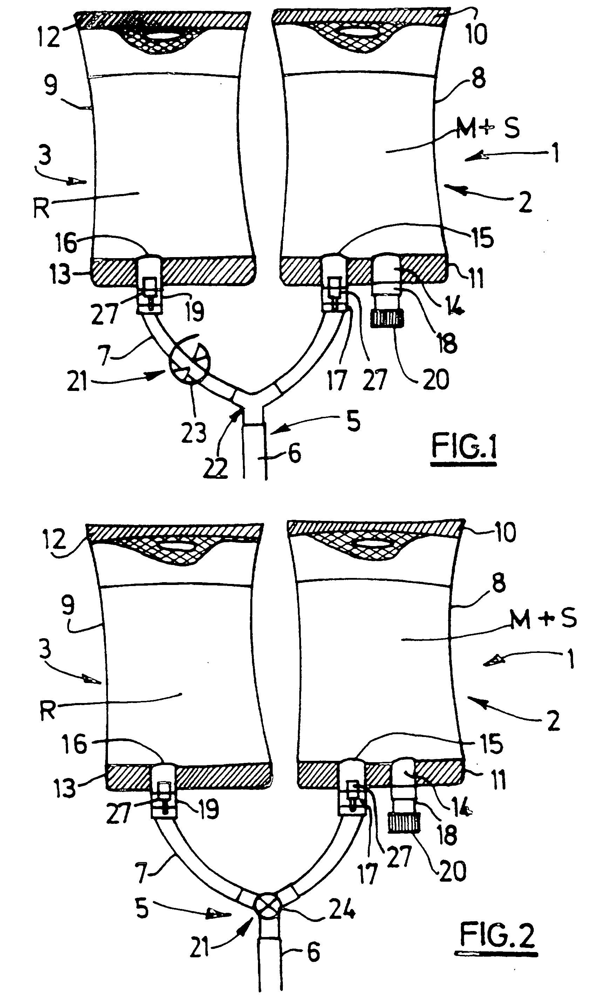

Les figures 1 à 4 sont des vues en élévation et à plat de quatre modes de réalisation du système de perfusion selon l'invention.

Un système de perfusion 1 va maintenant être décrit en référence à une position normale d'utilisation, dans lequel le contenu des poches 2, 3, 4 va être distribué, par exemple à un patient. Dans cette structure, les poches 2, 3, 4 sont suspendues par leurs parties extrêmes inférieures. Cette situation permet de définir les qualificatifs de « supérieur », « inférieur » et « latéral ».

Le système de perfusion 1 comprend des poches 2, 3, 4 et des moyens de distribution et de perfusion 5 associés aux dites poches pour former un ensemble unitaire en circuit fermé, en permanence.

Un premier type de poche, appelée par la suite poche de médicament 2, 4, est prévue pour contenir un produit M tel qu'un médicament en solution dans un soluté S.

Un deuxième type de poche, appelée par la suite poche de rinçage 3, est prévue pour contenir un soluté de rinçage ou de nutrition R des moyens de distribution et de perfusion 5. Dans un mode d'exécution, le soluté de rinçage R peut être le même que le soluté S.

Les moyens de distribution et de perfusion 5 comprennent essentiellement une tubulure 6 reliant les poches de médicament 2, 4 à un moyen de perfusion tel qu'un luer (non représenté).

La poche de rinçage 3 est associée aux moyens de distribution et de perfusion 5 en un ensemble unitaire, en circuit fermé. L'ensemble étant prévu pour perfuser à un patient, de façon sélective et successive, le contenu des poches de médicament 2, 4 et de rinçage 3.

Le système de perfusion 1 qui vient d'être décrit constitue un ensemble élémentaire qui peut être intégré à un système plus complexe, qui comprend par exemple d'autres poches, d'autres tubulures, des clamps ou des filtres, lesquels éléments ne seront pas décrits plus avant.

Les poches de médicament 2, 4 et de rinçage 3 comprennent chacune une enveloppe 8, 9, sous forme d'un tube aplati en matériau plastique souple, dont les parties extrêmes transversales 10, 11, 12, 13 ont été soudées transversalement.

Dans la réalisation particulière représentée sur les figures 1 à 3, l'enveloppe 8 de la poche de médicament 2, 4 comporte deux orifices 14, 15 et celle 9 de la poche de rinçage 3 un orifice 16 situé respectivement sur les même côtés transversaux des enveloppes 8, 9, à savoir supérieurs. Toutefois, les poches de médicament 2, 4 et de rinçage 3 peuvent être identiques et/ou comporter plus de deux orifices agencés différemment sur leurs enveloppes 8, 9 respectives.

Les orifices 14, 15, 16 sont de forme générale cylindrique, et sont munis chacun d'une portion de tube 17, 18, 19. Des systèmes sécables 27 peuvent être prévus à l'intérieur de ces portions de tube 17, 18, 19 de sorte à assurer, lors de leur rupture, la communication fluidique avec l'extérieur des enveloppes 8, 9 respectives.

Les moyens de distribution et de perfusion 5 sont montés de façon étanche dans une portion de tube 17 de la poche de médicament 2. La deuxième portion de tube 18 de la poche de médicament 2 est prévue pour recevoir un site d'injection permettant l'injection 20 du médicament M dans la poche de médicament 2, 4 au moyen d'une aiguille et d'une seringue ou d'un dispositif de transfert.

Dans un premier et deuxième mode de réalisation, représenté respectivement sur les figures 1 et 2, le système de perfusion 1 comprend une poche de médicament 2 et une poche de rinçage 3.

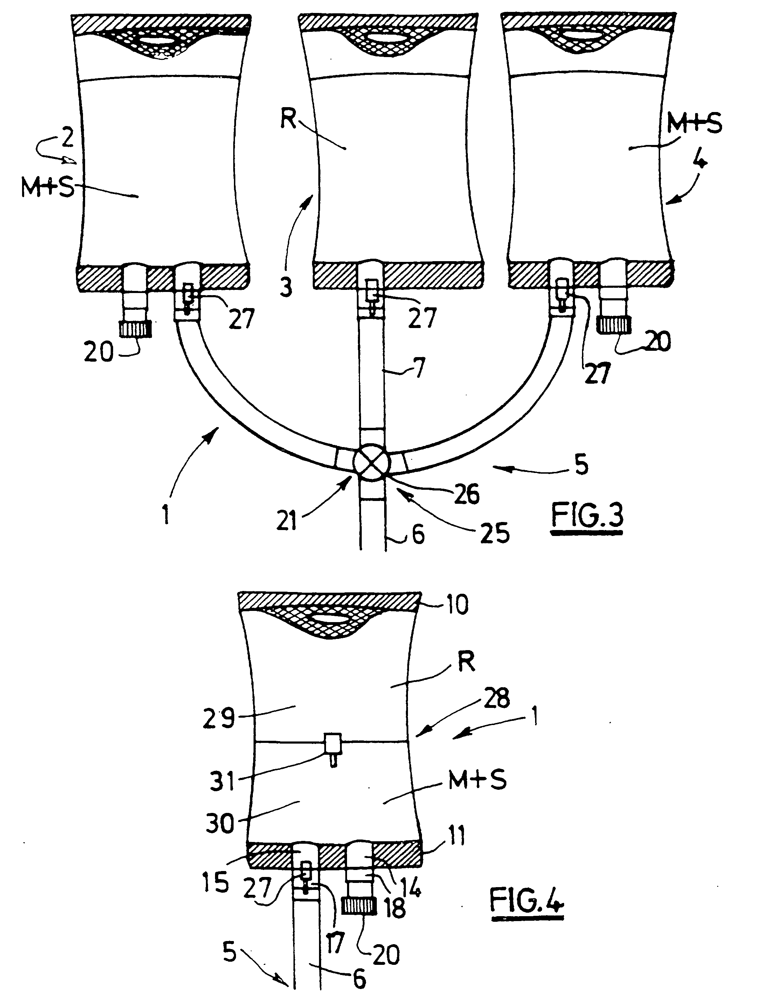

Dans un troisième mode de réalisation, représenté sur la figure 3, le système de perfusion 1 comprend deux poches de médicament 2, 4 et une seule poche de rinçage 3.

Dans un quatrième mode de réalisation représenté sur la figure 4, la poche de rinçage 3 est associée directement sur une poche de médicament 2, 4. Dans cette configuration, la poche de rinçage 3 et la poche de médicament 2,4 peuvent être réalisées sous la forme d'une seule poche 28 comprenant deux compartiments 29, 30 indépendants et séparés l'un de l'autre par un système sécable 31. Le compartiment inférieur 29 contenant le soluté de rinçage R et le compartiment supérieur 30, sur lequel sont montés les orifices de sortie14, 15, contenant le médicament M. Le système sécable 31 permet, lors de sa rupture, de mettre en communication fluidique le compartiment inférieur 29 avec le compartiment supérieur 30 et ainsi de permettre le passage du soluté de rinçage R dans les moyens de distribution et de perfusion 5 via le compartiment supérieur 30.

Dans les modes de réalisation représentés sur les figures 1 à 3, la poche de rinçage 3 est associée de façon unitaire en circuit fermé, par l'intermédiaire d'une tubulure 7, à l'ensemble constitué des poches de médicament 2, 4 et des moyens de distribution et de perfusion 5. Des moyens de sélection 21 sont prévus pour sélectionner le passage dans les moyens de distribution et de perfusion 5, soit du soluté S contenant le médicament M, soit du soluté de rinçage R.

Pour pouvoir effectuer un rinçage des moyens de distribution et de perfusion 5 le plus efficace possible, l'association de la poche de rinçage 3 au moyen de distribution et de perfusion 5 est réalisé à proximité des poches de médicament 2, 4.

Le mode d'association de la poche de rinçage 3 au moyen de perfusion et de distribution 5, ainsi que les moyens de sélection 21 peuvent faire l'objet de plusieurs variantes que nous allons décrire maintenant.

Dans une première variante (voir figure 1), l'association de la poche de médicament 2 sur les moyens de distribution et de perfusion 5, est réalisée par l'intermédiaire d'une jonction en Y 22. Les poches 2, 3 sont munies chacune d'un système sécable 27. Un clamp 23 est prévu sur la tubulure 7 associant la poche de rinçage 3 aux moyens de distribution et de perfusion 5.

Pour injecter le médicament M au patient, le système sécable 27 de la poche de médicament 2 est rompu. Lorsque la poche 2 est vide, le système sécable 27 de la poche de rinçage 3 est rompu à son tour pour permettre le rinçage des moyens de distribution et de perfusion 5. Le clamp 23 permet d'empêcher le passage du liquide de rinçage R dans les moyens de perfusion et de distribution 5, par exemple pour ne pas injecter la totalité du soluté de rinçage R au patient.

Dans une deuxième variante (voir figure 2), la connexion de la tubulure 7 associant la poche de rinçage 3 au moyen de distribution et de perfusion 5, est réalisée à l'aide d'un robinet à voies multiples 24, qui sert également, en combinaison avec les systèmes sécables 27 des poches 2, 3, de moyens de sélection 21.

Le robinet 24 est alors manoeuvré, après rupture du système sécable 27 de la poche 2, pour permettre la perfusion du médicament au patient puis, lorsque la poche de médicament 2 est vide, on rompt le système sécable de la poche 3 et on manoeuvre à nouveau les moyens de sélection 21 pour permettre le passage du liquide de rinçage R dans les moyens de distribution et de perfusion 5.

Dans une variante supplémentaire, représentée plus particulièrement sur la figure 3, en relation avec un système de perfusion comprenant deux poches de médicament 2, 4 et une poche de rinçage 3, un moyen de commutation 25 de la communication entre une poche de médicament 2, 4 et les moyens de distribution 5 et de perfusion est prévue.

Cette réalisation permet, notamment lorsque deux poches de médicament 2, 4 sont prévues, d'administrer sélectivement et successivement le contenu des poches de médicament 2, 4, en manoeuvrant les moyens de commutation 25. Le rinçage des moyens de distribution et de perfusion 5 pouvant être effectué, notamment lorsque la première poche de médicament est vide et avant d'administrer la deuxième dose de médicament, en manoeuvrant les moyens de sélection 21.

La figure 3 montre des moyens de sélection 21 et des moyens de commutation 25 formés par un même robinet à voies multiples 26 qui est manoeuvré pour permettre, après rupture des systèmes sécables correspondants, le passage, soit de la première dose de médicament, soit de la seconde dose de médicament, soit du liquide de rinçage.

Selon une autre variante, les moyens de sélection 21 et de commutation 25 sont distincts. Les moyens de commutation peuvent alors comprendre un clamp situé en amont de la connexion de la poche de rinçage 3 sur la tubulure 6 des moyens de distribution et de perfusion 5.

## Revendications

1. Système de perfusion (1) comprenant :
- au moins une poche (2, 4) - dite poche de médicament - destinée à contenir un certain produit tel qu'un médicament en solution dans un soluté ;
- des moyens de distribution et de perfusion (5) ;
- au moins une poche (3) - dite poche de rinçage - destinée à contenir un soluté pouvant servir de soluté de rinçage, ladite poche étant associée audit système de manière que le soluté de rinçage puisse passer dans les moyens de distribution et de perfusion (5) afin d'assurer leur rinçage ; et
- des moyens de sélection à commande externe (21), notamment manuelle, aptes à sélectionner le passage dans les moyens de distribution et de perfusion (5) du soluté contenant le médicament et/ou du soluté de rinçage ;
ledit système étant **caractérisé en ce que** les poches de médicament (2, 4) et de rinçage (3) sont associées au moyen de distribution et de perfusion (5) de sorte, avec les moyens de sélection (21), à former un ensemble unitaire en circuit fermé, en permanence.

2. Système de perfusion (1) selon la revendication 1, **caractérisé en ce que** la poche de rinçage (3) est associée par l'intermédiaire d'une tubulure (7) aux moyens de distribution et de perfusion (5).

3. Système de perfusion (1) selon la revendication 2, **caractérisé en ce que** les moyens de sélection (21) comprennent un clamp (23) positionné sur la tubulure (7) associant la poche de rinçage (3) aux moyens de distribution et de perfusion (5).

4. Système de perfusion (1) selon la revendication 2, **caractérisé en ce que** les moyens de sélection (21) comprennent un robinet à voies multiples (24, 26) réalisant la connexion entre la tubulure (7) associant la poche de rinçage (3) et les moyens de distribution et de perfusion (5).

5. Système de perfusion (1) selon l'une quelconques des revendications 1 à 4, **caractérisé en ce que** les moyens de sélection (21) comprennent des systèmes sécables (27) aptes, lors de leur rupture, à mettre la poche de rinçage (3) et/ou les poches de médicaments (2, 4) en communication fluidique avec les moyens de distribution et de perfusion (5).

6. Système de perfusion (1) selon la revendication 1, **caractérisé en ce que** la poche de rinçage est associée directement sur une poche de médicament (2, 4).

7. Système de perfusion (1) selon la revendication 6, **caractérisé en ce que** les moyens de sélection (21) comprennent un système sécable (31) apte, lors de sa rupture, à mettre en communication fluidique la poche de rinçage (3) avec la poche de médicament (2, 4).

8. Système de perfusion (1) selon l'une quelconque des revendications 1 à 7, **caractérisé en ce qu'**il comprend au moins deux poches de médicament (2, 4) destinées à recevoir deux doses de médicament identiques ou différentes, et au moins une poche de rinçage (3).

9. Système de perfusion (1) selon la revendication 8, **caractérisé en ce qu'**il comprend en outre un moyen de commutation (25) de la communication entre les poches de médicament (2, 4) et les moyens de distribution et de perfusion (5).

10. Procédé de mise en oeuvre d'un système de perfusion (1) selon l'une quelconque des revendications 1 à 9, dans lequel, après avoir perfusé le contenu de la poche de médicament (2, 4), on manoeuvre les moyens de sélection (21) pour permettre le rinçage des moyens de distribution et de perfusion (5) grâce au soluté de rinçage provenant de la poche de rinçage (3).

11. Procédé selon la revendication 10 pour la mise en oeuvre d'un système de perfusion (1) à deux poches de médicament (2, 4) et une poche de rinçage (3), dans lequel, après avoir perfusé le contenu de la première poche de médicament, on manoeuvre les moyens de sélection (21) pour permettre le rinçage des moyens de distribution et de perfusion (5) avec une partie du contenu de la poche de rinçage (3), puis on manoeuvre les moyens de sélection (21) et les moyens de commutation (25) pour perfuser le contenu de la deuxième poche de médicament, enfin, lorsque cette deuxième poche est vide, on manoeuvre les moyens de sélection (21) pour permettre le rinçage des moyens de distribution et de perfusion (5) avec le reste du contenu de la poche de rinçage (3).

## Patentansprüche

1. Infusionssystem (1) bestehend aus :
- mindestens einem Beutel (2, 4) - dem so genannten Medikamentbeutel - zur Aufnahme eines bestimmten Produkts wie beispielsweise eines Medikaments in medikamentöser Lösung;
- Abgabe- und Infusionsmitteln (5);
- mindestens einem Beutel (3) - dem so genannten Spülbeutel - zur Aufnahme einer medikamentösen Lösung, die als Spüllösung verwendet werden kann, wobei dieser Beutel so mit dem System verbunden ist, dass die Spüllösung in die Abgabe- und Infusionsmittel (5) gelangen kann, um deren Spülung zu gewährleisten; und
- extern, insbesondere manuell gesteuerten Auswahlmitteln (21) zur Auswahl des Einfließens der medikamentösen Lösung und/oder der Spüllösung in die Abgabe- und Infusionsmittel (5);
wobei das System **dadurch gekennzeichnet ist, dass** die Medikamentbeutel (2, 4) und die Spülbeutel (3) derart mit den Abgabe- und Infusionsmitteln (5) verbunden sind, dass sie eine einzige Einheit in permanent geschlossenem Kreis bilden.

2. Infusionssystem (1) nach Anspruch 1, **dadurch gekennzeichnet, dass** der Spülbeutel (3) über einen Stutzen (7) mit den Abgabe- und Infusionsmitteln (5) verbunden ist.

3. Infusionssystem (1) nach Anspruch 2, **dadurch gekennzeichnet, dass** die Auswahlmittel (21) einen Clamp (23) umfassen, der auf dem den Spülbeutel (3) mit den Abgabe- und Infusionsmitteln (5) verbindenden Stutzen (7) angeordnet ist.

4. Infusionssystem (1) nach Anspruch 2, **dadurch gekennzeichnet, dass** die Auswahlmittel (21) ein Mehrwegeventil (24, 26) umfassen, dass den Anschluss zwischen dem den Spülbeutel (3) mit den Abgabe- und Infusionsmitteln (5) verbindenden Stutzen (7) herstellt.

5. Infusionssystem (1) nach einem der Ansprüche 1 bis 4, **dadurch gekennzeichnet, dass** die Auswahlmittel (21) trennbare Systeme (27) umfassen, die bei ihrem Bruch den Spülbeutel (3) und/oder die Medikamentbeutel (2, 4) mit den Abgabe- und Infusionsmitteln (5) in Flüssigkeitsverbindung setzen können.

6. Infusionssystem (1) nach Anspruch 1, **dadurch gekennzeichnet, dass** der Spülbeutel unmittelbar mit einem Medikamentbeutel (2, 4) verbunden ist.

7. Infusionssystem (1) nach Anspruch 6, **dadurch gekennzeichnet, dass** die Auswahlmittel (21) ein trennbares System (31) umfassen, das bei seinem Bruch den Spülbeutel (3) mit dem Medikamentbeutel (2, 4) in Flüssigkeitsverbindung setzen kann.

8. Infusionssystem (1) nach einem der Ansprüche 1 bis 7, **dadurch gekennzeichnet, dass** es mindestens zwei Medikamentbeutel (2, 4) umfasst für die Aufnahme von zwei identischen oder verschiedenen Medikamentdosen, sowie mindestens einen Spülbeutel (3).

9. Infusionssystem (1) nach Anspruch 8, **dadurch gekennzeichnet, dass** es ferner ein Schaltmittel (25) für die Verbindung zwischen den Medikamentbeuteln (2, 4) und den Abgabe- und Infusionsmitteln (5) umfasst.

10. Verfahren für den Einsatz eines Infusionssystems (1) nach einem der Ansprüche 1 bis 9, bei dem man nach Infusion des Inhalts des Medikamentbeutels (2, 4) die Auswahlmittel (21) betätigt, um das Spülen der Abgabe- und Infusionsmittel (5) mit der aus dem Spülbeutel (3) kommenden Spüllösung zu ermöglichen.

11. Verfahren nach Anspruch 10 für den Einsatz eines Infusionssystems (1) mit zwei Medikamentbeuteln (2, 4) und einem Spülbeutel (3), bei dem man nach Infusion des Inhalts des ersten Medikamentbeutels die Auswahlmittel (21) betätigt, um das Spülen der Abgabe- und Infusionsmittel (5) mit einem Teil des Inhalts des Spülbeutels (3) zu ermöglichen, und man die Auswahlmittel (21) und die Schaltmittel (25) betätigt, um den Inhalt des zweiten Medikamentbeutels zu übertragen, und, wenn dieser zweite Beutel leer ist, man die Auswahlmittel (21) betätigt, um das Spülen der Abgabe- und Infusionsmittel (5) mit dem Rest des Inhalts des Spülbeutels (3) zu ermöglichen.

## Claims

1. A perfusion system (1) comprising:
- at least one pocket (2, 4) - referred to as a medicine pocket - intended to contain a certain product such as a medicine in solution in a solute;
- dispensing and perfusion means (5);
- at least one pocket (3) - referred to as a rinsing pocket
- intended to contain a solute capable of serving as a rinsing solute, said pocket being associated with said system so that the rinsing solute can pass into the dispensing and perfusion means (5) in order to provide their rinsing; and
- selection means (21) with external control, in particular manual control, able to select the passage into the dispensing and perfusion means (5) of the solute containing the medicine and/or of the rinsing solute;
said system being **characterised in that** the medicine pockets (2, 4) and the rinsing pocket (3) are associated with the dispensing and perfusion means (5) so as to form, with the selection means (21), a closed-circuit unitary assembly, permanently.

2. A perfusion system (1) according to Claim 1, **characterised in that** the rinsing pocket (3) is associated by means of a connection piece (7) with the dispensing and perfusion means (5).

3. A perfusion system according to Claim 2, **characterised in that** the selection means (21) comprise a clamp (23) positioned on the connection piece (7) associating the rinsing pocket (3) with the dispensing and perfusion means (5).

4. A perfusion system (1) according to Claim 2, **characterised in that** the selection means (21) comprise a multi-way valve (24, 26) implementing the connection between the connection piece (7) associating the rinsing pocket (3) and the dispensing and perfusion means (5).

5. A perfusion system (1) according to any one of Claims 1 to 4, **characterised in that** the selection means (21) comprise systems (27) that can be cut, able, when severed, to put the rinsing pocket (3) and/or the medicine pockets (2, 4) in fluidic communication with the dispensing and perfusion means (5).

6. A perfusion system (1) according to Claim 1, **characterised in that** the rinsing pocket is associated directly on a medicine pocket (2, 4).

7. A perfusion system (1) according to Claim 6, **characterised in that** the selection means (21) comprise a system (31) that can be cut, able, when severed, to put the rinsing pocket (3) into fluidic communication with the medicine pocket (2, 4).

8. A perfusion system (1) according to any one of Claims 1 to 7, **characterised in that** it comprises at least two medicine pockets (2, 4) intended to receive two identical or different doses of medicine, and at least one rinsing pocket (3).

9. A perfusion system (1) according to Claim 8, **characterised in that** it also comprises a means (25) of switching the communication between the medicine pockets (2, 4) and the dispensing and perfusion means (5).

10. A method of implementing a perfusion system (1) according to any one of Claims 1 to 9, in which, after having perfused the contents of the medicine pocket (2, 4), the selection means (21) are operated in order to allow the rinsing of the dispensing and perfusion means (5) using the rinsing solute coming from the rinsing pocket (3).

11. A method according to Claim 10 for the implementation of a perfusion system (1) with two medicine pockets (2, 4) and one rinsing pocket (3), in which, after having perfused the contents of the first medicine pocket, the selection means (21) are operated in order to allow the rinsing of the dispensing and perfusion means (5) with part of the contents of the rinsing pocket (3), then the selection means (21) and the switching means (25) are operated in order to perfuse the contents of the second medicine pocket, and finally, when this second pocket is empty, the selection means (21) are operated in order to allow the rinsing of the dispensing and perfusion means (5) with the rest of the contents of the rinsing pocket (3).
